(19) Europäisches Patentamt
European Patent Office
Office européen des brevets

(11) Veröffentlichungsnummer: **0 387 193 A1**

# (12) EUROPÄISCHE PATENTANMELDUNG

(21) Anmeldenummer: **90810149.6**

(22) Anmeldetag: **27.02.90**

(51) Int. Cl.5: **C07D 209/76, C08G 73/12, C08F 22/40, G03F 7/027**

(30) Priorität: **07.03.89 CH 829/89**

(43) Veröffentlichungstag der Anmeldung: **12.09.90 Patentblatt 90/37**

(84) Benannte Vertragsstaaten: **DE ES FR GB IT NL SE**

(71) Anmelder: **CIBA-GEIGY AG**
**Klybeckstrasse 141**
**CH-4002 Basel(CH)**

(72) Erfinder: **Renner, Alfred, Dr.**
**Marcoup 2**
**CH-3286 Muntelier(CH)**
Erfinder: **Vonlanthen, Christian**
**La Verdure**
**CH-1711 Ependes(CH)**
Erfinder: **Irving, Edward, Dr.**
**Applecroft, Dark Lane**
**Higher Whitley, Warrington WA4 4QF(GB)**
Erfinder: **Banks, Christopher Paul, Dr.**
**105, Ross Close**
**Saffron Walden, Essex CB11 4DU(GB)**

## (54) Substituierte ungesättigte bireaktive bicyclische Imide und deren Verwendung.

(57) Imide der Formel I

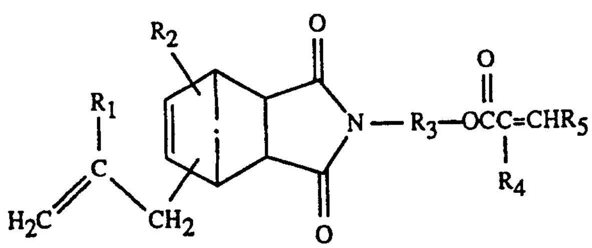

(I),

worin $R_1$, $R_2$ und $R_4$ unabhängig voneinander Wasserstoff oder Methyl bedeuten, $R_3$ eine direkte Bindung oder ein gegebenenfalls durch O-Atome unterbrochener $C_2$-$C_{20}$-aliphatischer, ein ein- oder mehrkerniger $C_5$-$C_{20}$-cycloaliphatischer oder $C_6$-$C_{20}$-aromatischer Rest ist oder für eine Gruppe der Formel II steht

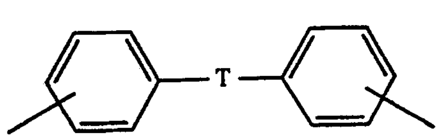

(II),

worin T Methylen, Isopropyliden, CO, O, S oder $SO_2$ bedeutet, und $R_5$ Wasserstoff oder Phenyl ist, können sowohl photochemisch als auch thermisch vernetzt werden und ergeben Polymere mit ausgezeichneten Eigenschaften, insbesondere einer sehr hohen Hitzebeständigkeit. Sie eignen sich insbesondere für die Herstellung von hitzebeständigen Photolithographien oder als Matrixharze für die Herstellung von Verbundstoffen.

EP 0 387 193 A1

## Substituierte ungesättigte bireaktive bicyclische Imide und deren Verwendung

Die Erfindung betrifft mit Allyl- oder Methallylgruppen sowie mit Acryloyloxy-, Methacryloyloxy- oder Cinnamoyloxygruppen substituierte Bicyclo[2.2.1]hept-5-en-2,3-dicarbonsäureimide, deren Herstellung, die daraus durch Erwärmen oder durch Photopolymerisation erhältlichen Polymeren sowie die Verwendung der besagten Imide als Photoresists oder als Matrixharze zur Herstellung von Prepregs.

Die U.S. 4,515,962 und U.S. 4,742,166 beschreiben allyl- oder methallyl-substituierte Bicyclo[2.2.1]hept-5-en-2,3-dicarbonsäureimide und die daraus durch Erhitzen erhältlichen Polymere.

Die U.S. 4,728,742 beschreibt allyl- oder methallyl-substituierte hydroxylgruppen-haltige Bicyclo[2.2.1]-hept-5-en-2,3-dicarbonsäureimide sowie die daraus gegebenenfalls in Anwesenheit anderer Comonomeren durch Erhitzen erhältlichen Polymere.

Die U.S. 4,709,047 beschreibt allyl- oder methallyl-substituierte sulfonyloxygruppenhaltige Bicyclo-[2.2.1]hept-5-en-2,3-dicarbonsäureimide und deren Verwendung als Katalysatoren für die thermische Vernetzung von kationisch polymerisierbaren Monomeren.

Die U.S. 4,440,850 beschreibt ein Verfahren zur Bilderzeugung, in dem als Photoresist Verbindungen eingesetzt werden, welche im selben Molekül sowohl eine Acryloyl- oder Methacryloylgruppe als auch eine gegebenenfalls substituierte Bicyclo[2.2.1]hept-5-en-2-yl- oder 7-Oxabicyclo[2.2.1]hept-5-en-2-yl-Gruppe enthalten. Dabei kann es sich um Ester der Bicycloheptenmonocarbonsäure, um Mono- oder Diester der Bicycloheptendicarbonsäure als auch um Imide der letztgenannten Säure handeln. Die Bicycloheptenylgruppen können unsubstituiert sein oder eine bis vier Methylgruppen oder eine Allylgruppe als Substituenten enthalten.

Allylsubstituierte Bicyclo[2.2.1]hept-5-en-2,3-dicarbonsäureimide sind in der U.S. 4,440,850 nicht spezifisch offenbart. Die im Verfahren gemäss der U.S. 4,440,850 verwendeten Verbindungen werden ausschliesslich durch Einwirkung aktinischer Strahlung polymerisiert. Eine Polymerisation durch Erhitzen dieser Verbindungen ist dabei nicht erwähnt.

Gegenstand vorliegender Erfindung sind Imide der Formel I

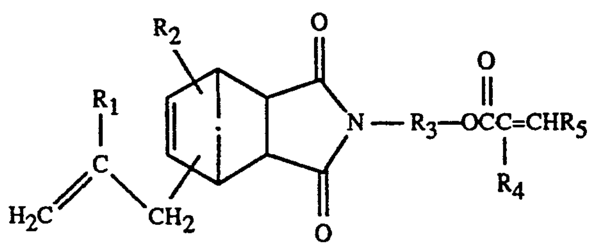

(I),

worin $R_1$, $R_2$ und $R_4$ unabhängig voneinander Wasserstoff oder Methyl bedeuten, $R_3$ eine direkte Bindung oder ein gegebenenfalls durch O-Atome unterbrochener $C_2$-$C_{20}$-aliphatischer, ein ein- oder mehrkerniger $C_5$-$C_{20}$-cycloaliphatischer oder $C_6$-$C_{20}$-aromatischer Rest ist oder für eine Gruppe der Formel II steht

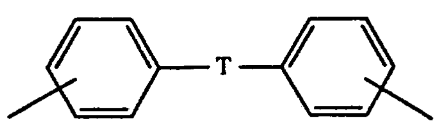

(II),

worin T Methylen, Isopropyliden, CO, O, S oder $SO_2$ bedeutet, und $R_5$ Wasserstoff oder Phenyl ist.

Ueberraschenderweise können die erfindungsgemässen Verbindungen trotz der Anhäufung sehr reaktiver Doppelbindungen durch Destillation rein dargestellt und ohne vorzeitige Gelierung verarbeitet werden.

Die erfindungsgemässen Imide sind bireaktive Monomere, welche sowohl durch Einwirken aktinischer Strahlung als auch thermisch polymerisiert werden können. Die so erhaltenen vernetzten Polymeren zeichnen sich insbesondere durch eine hohe Glasumwandlungstemperatur und eine grosse Hitzebeständigkeit aus. Gegenstand der Erfindung sind somit auch Polymere, die dadurch erhältlich sind, dass man ein Imid der Formel I während 6 bis 60 Stunden auf eine Temperatur zwischen 180 und 300 °C, vorzugsweise zwischen 200 und 250 °C erhitzt, sowie Polymere, die dadurch erhältlich sind, dass man ein Imid der

Formel I, gegebenenfalls in Gegenwart eines Photoinitiators, mit aktinischer Strahlung belichtet.

Unter den erfindungsgemässen Imiden der Formel I werden solche bevorzugt, worin $R_1$ und $R_2$ je Wasserstoff bedeuten.

Der Rest

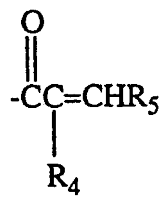

der erfindungsgemässen Imide stammt von der entsprechenden ungesättigten Carbonsäure, vorzugsweise Zimtsäure, Methacryl- oder insbesondere Acrylsäure ab. Somit werden Imide der Formel I bevorzugt, worin $R_4$ Wasserstoff und $R_5$ Phenyl bedeuten, oder Imide, worin $R_5$ Wasserstoff und $R_4$ Methyl oder vorzugsweise Wasserstoff bedeuten.

$R_3$ kann ein zweiwertiger geradkettiger oder verzweigter aliphatischer Rest mit 2-20, vorzugsweise 2-10 und insbesondere 2-6 C-Atomen sein, der in der Kette durch ein oder mehrere Sauerstoffatome unterbrochen sein kann. Beispiele für geeignete aliphatische Reste $R_3$ sind Ethylen, 1,2- und 1,3-Propylen, Butylen, Penta- und Hexamethylen, Heptylen, Octylen Decylen, Dodecylen, Hexadecylen oder Neopentylen. Durch Sauerstoffatome unterbrochene aliphatische Reste können beispielsweise von Ethylenglykol oder von 1,2- oder 1,3-Propylenglykol abgeleitet sein und können Gruppen der Formeln

$-CH_2CH_2-[-OCH_2CH_2-]_m$ , 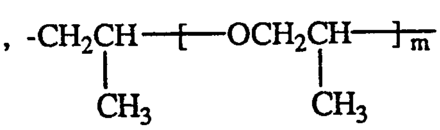 oder

$-CH_2CH_2CH_2-[-OCH_2CH_2CH_2-]_m$ mit m = 1-8 entsprechen.

Wenn $R_3$ von Ethylenglykol abgeleitet ist, bedeutet m geeigneterweise 1-8, insbesondere 1-4. Falls $R_3$ von Propylenglykol abgeleitet ist, bedeutet m z.B. 1-5, insbesondere 1-3.

$R_3$ kann auch ein ein- oder mehrkerniger cycloaliphatischer zweiwertiger Rest mit 5-20 C-Atomen sein, wie beispielsweise Cyclopentylen, Cyclohexylen, Cycloheptylen, Cyclooctylen, Bis(cyclohexylen)methan, 2,2-Bis(cyclohexylen)propan und Decalinylen. Besonders bevorzugt ist Cyclohexylen, insbesondere 1,4-Cyclohexylen.

Wenn $R_3$ einen aromatischen Rest bedeutet, so handelt es sich vorzugsweise um 1,3- oder 1,4-Phenylen oder Naphthylen, die jeweils, falls gewünscht, auch durch eine oder mehrere $C_{1-4}$-Alkylgruppen, wie Methyl, Ethyl oder Propyl, substituiert sein können. Bevorzugt sind die genannten Gruppen unsubstituiert. 1,3- und 1,4-Phenylengruppen sind als aromatische Reste besonders bevorzugt.

Wenn $R_3$ eine Gruppe der Formel II bedeutet, steht T vorzugsweise für O, $SO_2$, Methylen oder Isopropyliden.

Besonders bevorzugte Imide der Formel I sind solche, worin $R_3$ eine direkte Bindung, ein $C_2$-$C_{10}$-aliphatischer Rest oder eine Gruppe

$-CH_2CH_2-[-OCH_2CH_2-]_m$ oder 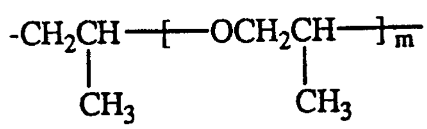

mit m = 1 oder 2, ein $C_5$-$C_6$-cycloaliphatischer oder $C_6$-$C_{10}$-aromatischer Rest ist oder für eine Gruppe der Formel II steht, worin T Methylen oder Isopropyliden bedeutet.

Ganz besonders bevorzugt sind Imide der Formel I, worin $R_3$ eine direkte Bindung, den Rest

$-CH_2CH_2-$, 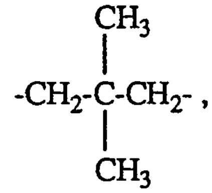, $-CH_2CH_2OCH_2CH_2-$,

1,3- oder 1,4-Cyclohexylen oder 1,3-oder 1,4-Phenylen bedeutet.

Am meisten bevorzugt sind Imide der Formel I, worin $R_1$, $R_2$ und $R_5$ je Wasserstoff, $R_3$ eine direkte Bindung oder der Rest $-CH_2CH_2-$ und $R_4$ Wasserstoff oder Methyl sind, und insbesondere das Imid der Formel I, worin $R_1$, $R_2$, $R_4$ und $R_5$ je Wasserstoff bedeuten und $R_3$ der Rest $-CH_2CH_2-$ ist.

Die erfindungsgemässen Imide können auf an sich bekannte Weise, z.B. durch Umsetzung eines hydroxylgruppen-haltigen Imids der Formel III

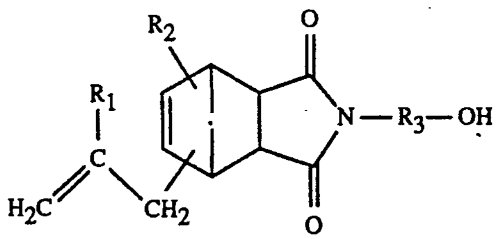

(III),

mit einer Verbindung der Formel IV hergestellt werden

$$R_6\overset{\overset{\displaystyle O}{\|}}{C}CR_4=CHR_5 \quad (IV),$$

wobei die Reste $R_1$, $R_2$, $R_3$, $R_4$ und $R_5$ die oben angegebene Bedeutung haben und $R_6$ für OH, Halogen oder $C_1$-$C_4$-Alkoxy steht.

Verbindungen der Formel III und deren Herstellung sind in der U.S. 4,728,742 beschrieben. Verbindungen der Formel IV sind bekannt und im Handel erhältlich. Wenn $R_6$ Halogen bedeutet, werden die entsprechenden Säurebromide und insbesondere -chloride bevorzugt. Ist $R_6$ eine Alkoxygruppe $OR_7$, handelt es sich vorzugsweise um den Methylester.

Geeignete Reaktionsbedingungen für die Veresterung von Alkoholen oder Phenolen mit esterbildenden Säurederivaten, wie den Verbindungen der Formel IV, sind dem Fachmann bekannt. Werden bei der Veresterung der Verbindungen der Formel III freie Carbonsäuren oder Carbonsäureester der Formel IV eingesetzt, so wird das Reaktionsgemisch vorzugsweise unter Rückfluss erhitzt, wobei das bei der Umsetzung entstehende Wasser oder der Alkohol während der Reaktion, gegebenenfalls als Azeotrop mit dem Lösungsmittel, abdestilliert werden.

Wird die Veresterung des hydroxylgruppen-haltigen Imids der Formel III mit einem Säurehalogenid der Formel IV vorgenommen, so führt man die Umsetzung vorzugsweise unter Kühlung in Gegenwart eines Säureakzeptors, beispielsweise in Gegenwart von tertiären Aminen, wie z.B. Triethylamin, Pyridin oder N,N-Dimethylanilin, durch. Die letztgenannte Umsetzung der Imide der Formel III mit Säurehalogeniden, insbesondere Säurechloriden, der Formel IV wird als Herstellungsverfähren der erfindungsgemässen Verbindungen bevorzugt.

Die erfindungsgemässen Verbindungen sind flüssige oder niedrigschmelzende feste Stoffe, welche sich durch hohe Reaktivität und, wegen ihrer niedrigen Viskosität, durch leichte Verarbeitung auszeichnen und welche zu festen Produkten mit hoher Glasumwandlungstemperatur polymerisiert werden können. Die Polymere weisen eine sehr hohe Hitzebeständigkeit auf und zeigen einen sehr geringen Schwund bzw. einen geringen Gewichtsverlust bei hoher Temperatur.

Dank der Anwesenheit von zwei verschiedenartigen reaktiven Gruppen, der olefinischen Doppelbindungen sowie der Acryloyl-, Methacryloyl- oder Cinnamoylgruppen, können die erfindungsgemässen Imide als Edukte bzw. Zwischenprodukte für die Herstellung verschiedenartiger Polymeren verwendet werden. Wegen ihrer niedrigen Viskosität eignen sich die erfindungsgemässen Imide auch als polymerisierbare Lösungsmittel bzw. als reaktive Verdünner für ungesättigte Imidharze. Selbstverständlich können den Imiden der Formel I inerte und stabile Stoffe, wie Füllmittel, Pigmente, Farbstoffe und andere Additive zugegeben werden, bevor sie zu vemetzten Gebilden polymerisiert werden.

Die erfidungsgemässen Verbindungen können direkt verwendet und polymerisiert werden, oder sie können zunächst in einem organischen Lösungsmittel, wie Toluol, Xylol, Methylethylketon, Ethylenglykolmonoalkyl- und -dialkylethern mit 1-4 C-Atomen in den Alkylgruppen oder einem ähnlichen üblichen Lösungsmitttel, gelöst werden. Solche Lösungen können als Imprägniermittel oder Beschichtungsmittel oder auch als Versandobjekt an den Verbraucher dienen.

Die erfindungsgemässen Imide können, falls zweckmässig, auch in Gegenwart anderer copolymerisierbarer Verbindungen polymerisiert werden. Dafür besonders geeignet sind andere ungesättigte Imide, insbesondere die substituierten ungesättigten bicyclischen Imide gemäss der U.S. 4,515,962 und der U.S. 4,742,166. Weitere geeignete copolymerisierbare Verbindungen sind solche, die zwei oder mehrere Acryloyl- oder Methacryloylgruppen enthalten, wie beispielsweise Umsetzungsprodukte von gegebenenfalls vorverlängerten Epoxidharzen mit stöchiometrischen Mengen Acryl oder Methacrylsäure, so dass das Produkt im wesentlichen keine freien Epoxidgruppen mehr enthält. Solche Produkte werden z.B. von der DOW Chemical Company unter dem Handelsnamen Derakane® verkauft.

Falls zweckmässig, können den erfindungsgemässen Imiden bzw. diese Imide enthaltenden Gemischen auch Härtungskatalysatoren beigegeben werden. Als Katalysatoren für die thermische Vernetzung besonders geeignet sind die in der U.S. 4,709,047 beschriebenen allyl- oder methallyl-substituierten sulfonyloxygruppen-haltigen ungesättigten bicyclischen Imide, insbesondere das Allylbicyclo[2.2. 1]hept-5-en-2,3-dicarbonsäure-N-benzolsulfonyloxyimid. Weitere geeignete Katalysatoren für die thermische Vernetzung sind z.B. Benzoylperoxyd, t-Butyl-perbenzoat, Cumolhydroperoxyd, Di-t-butylperoxyd, Bis-t-butylperoxy-butan, Methylethylketon-Hydroperoxyd und Dicumylperoxyd. Die Konzentration des allfällig zu verwendenden Katalysators liegt zweckmässig zwischen 0,1 und 15,0, vorzugsweise zwischen 0,25 und 5,0, besonders bevorzugt zwischen 0,5 und 2,0 Gew.%, bezogen auf das Gesamtgewicht des Imids der Formel I und der gegebenenfalls im Gemisch enthaltenen anderen ungesättigten Imide.

Bei der photochemischen Polymerisation der erfindungsgemässen Imide verwendet man vorzugsweise geeignete Photoinitiatoren als Katalysator. Beispiele für geeignete Photoinitiatoren sind α-halogensubstituierte Acetophenone wie Trichlormethyl-4′-tert.-butylphenylketon, α-hydroxy-α-alkyl-substituierte Acetophenone wie 2-Hydroxy-2-methyl-1-phenyl-propanon-1, 1-Hydroxycyclohexylphenylketon, Benzoin und dessen Alkylether (z.B. der n-Butylether), α-Methylbenzoin, α,α-Dialkoxy-α-benzoylessigsäurealkylester, Benzophenone wie Benzophenon selbst und 4,4′-Bis(dimethylamino)benzophenon, O-Alkoxycarbonylderivate eines Oxims des Benzils oder 1-Phenylpropan-1,2-dions, wie Benzil(O-ethoxycarbonyl)-α-monoxim und 1-Phenylpropan-1,2-dion-2-(O-ethoxycarbonyl)oxim, Benzilketale, z.B. dessen Dimethylketal, substituierte Thioxanthone, z.B. 2-Chlorthioxanthon, Antrachinone, Ester der Phenylglyoxylsäure, 2-Benzoyl-2-phenyl-1,3-dioxolane und 4-Benzoyl-4-phenyl-1,3-dioxolane sowie Photoredoxsysteme, die aus einem Gemisch aus einem Phenothiazinfarbstoff (z.B. Methylenblau) oder einem Chinoxalin (z.B. einem Metallsalz der 2-(m- oder p-methoxyphenyl)-chinoxalin-6′- oder -7′-sulfonsäure) mit einem Elektronendonator wie Benzolsulfinsäure oder einer anderen Sulfinsäure oder einem Salz davon, z.B. dem Natriumsalz, oder einem Arsin, einem Phosphin oder Thioharnstoff bestehen.

Geeignete Photoinitiatoren sind auch α-Aminoacetophenonderivate wie z.B. 2-Methyl-1-[4-(methylthio)-phenyl]-2-morpholinopropan-1-on. Solche Verbindungen sind beispielsweise in der U.S. 4,318,791, U.S. 4,582,862 und U.S. 4,739,052 beschrieben. Bevorzugte Photoinitiatoren sind Benzildimethylketal, 1 Hydroxycyclohexylphenylketon und 2-Methyl-1-[4(methylthio)phenyl]-2-morpholinopropan-1-on. Im allgemeinen werden etwa 0,15 bis 10 Gew.-%, vorzugsweise etwa 2,5 bis 5 Gew.-% Photoinitiator zugegeben, bezogen auf das Gesamtgewicht der erfindungsgemässen Imide und der gegebenenfalls im Gemisch enthaltenen allfälligen anderen Verbindungen, welche Acryloyl- oder Methacryloylgruppen aufweisen.

Wegen der hohen Reaktivität der erfindungsgemässen Imide und angesichts der Tatsache, dass es sich dabei um bireaktive Monomere handelt, eignen sich diese Verbindungen für den Einsatz bei einer Vielzahl von Anwendungen, wie z.B. als Laminier- oder Elektroharze, als Hochtemperatur-Klebstoffe oder zur Herstellung von Beschichtungen oder Formkörpern, Prepregs und Verbundstoffen. Für viele Anwendungen kann es dabei von Vorteil sein, die flüssigen Verbindungen bzw. Gemische zuerst unter Einwirkung aktinischer Strahlung zu verfestigen und anschliessend thermisch zu vernetzten Polymeren reagieren zu lassen.

Ein bevorzugtes Anwendungsgebiet der Imide der Formel I ist deren Verwendung als Photoresists.

Gegenstand der Erfindung ist daher auch ein Verfahren zur Bilderzeugung, dadurch gekennzeichnet, dass man eine auf einem Träger aufgebrachte Schicht enthaltend ein Imid der Formel I in vorbestimmtem Muster bildmässig mit aktinischer Strahlung belichtet, so dass die belichteten Stellen der Schicht photopolymerisierten werden, und anschliessend die Abbildung durch Auflösen der nicht photopolymerisierten Stellen der Schicht in einem Lösungsmittel entwickelt.

Falls gewünscht, kann das entstehende Bild nach der Entwicklung noch thermisch nachbehandelt

werden, beispielsweise durch Erhitzen während 1-5 Stunden auf 100-200 °C. Dadurch werden besonders hitzebeständige Photolithographien erhalten, bei denen der Gewichtsverlust erst bei Temperaturen von über 400 °C einsetzt. Die flüssigen Imide der Formel I bzw. die flüssigen sie enthaltende Zusammensetzungen lassen sich nach herkömmlichen Methoden wie Sprühbeschichtung, Aufschleudern, Walzenauftrag, Kaskadenbeschichtung und insbesondere Vorhangbeschichtung auf geeignete Träger aufbringen. Typischerweise wird der Träger so beschichtet, dass die Schicht 1 bis 250 µm, vorzugsweise 10 bis 30 µm, dick ist. Der Träger kann beispielsweise aus Kupfer, Aluminium oder einem anderen Metall, aus Papier, Kunstharz oder Glas bestehen.

Bei der Photopolymerisationsstufe des erfindungsgemässen Verfahrens verwendet man bevorzugt aktinische Strahlung einer Wellenlänge von 200-600 nm. Als aktinische Strahlungsquellen eignen sich unter anderem Kohlelichtbögen, Quecksilberdampflicht bögen, Leuchtröhren mit ultraviolettes Licht ausstrahlenden Leuchtstoffen, Argon und Xenonglühlampen, Wolframlampen und photographische Flutlichtlampen. Darunter sind Quecksilberdampflichtbögen, insbesondere Höhensonnen, fluoreszierende Höhensonnen und Metallhalogenidlampen am besten geeignet. Die zur Belichtung der Imide der Formel I bzw. der sie enthaltenden Zusammensetzung erforderlichen Zeiten hängen von verschiedenen Faktoren ab, unter anderem beispielsweise den einzelnen verwendeten Verbindungen, der Art der Lichtquelle und deren Abstand von der bestrahlten Schicht. Der mit Photopolymerisationsmethoden vertraute Fachmann kann die geeigneten Zeiten leicht bestimmen. Beispielsweise kann das Imid oder die Zusammensetzung im Abstand von 10-100 cm von einer Strahlungsquelle 1-10 Minuten lang bestrahlt werden.

Zum Entwickeln der Abbildung geeignete Lösungsmittel lassen sich leicht durch Serienversuche auffinden: diese sind unter anderem Cyclohexanon, Trimethylcyclohexanon, Ethanol, Toluol, 2-Ethoxyethanol, 1,1,1 -Trichlorethan und deren Gemische. Es kann notwendig sein, die Wirkung des Lösungsmittels durch Rühren oder leichtes Bürsten zu unterstützen. Besitzt der Träger eine Schicht eines geeigneten elektrisch leitenden Metalls, üblicherweise Kupfer oder Silber, in direkter Berührung mit der photopolymerisierten Zusammensetzung, so kann das unvernetzte Polymer gegebenenfalls entfernt werden, um das Metall freizulegen. So freigelegtes Metall kann dann an den unbelichteten Stellen mittels Aetzflüssigkeiten wie Ferrichlorid- oder Ammoniumpersulfatlösungen weggeätzt werden, um eine gedruckte Schaltung zu bilden.

Ein weiteres bevorzugtes Anwendungsgebiet der erfindungsgemässen Imide ist deren Einsatz als Matrixharze für Composites. Gegenstand der Erfindung ist somit auch die Verwendung der Imide der Formel I zur Herstellung von Prepregs sowie ein Verfahren zur Herstellung von Prepregs, dadurch gekennzeichnet, dass man ein faserartiges Material mit einem Imid der Formel I oder mit einem Gemisch enthaltend ein Imid der Formel I imprägniert und anschliessend das imprägnierte Material aktinischer Strahlung aussetzt, so dass sich das Imid oder das imidhaltige Gemisch durch Photopolymerisation verfestigt und im wesentlichen klebfrei wird, aber noch thermisch vernetzbar bleibt.

Geeignetes faserartiges Material sind beispielsweise Glasfaser-, Kohlenstoffaser- oder Aramidfaser-Gewebe, wie z.B. Fasergewebe aus den unter dem Handelsnamen Kevlar® bekannten Poly(1,4-phenylenterephthalamiden).

Die folgenden Beispiele erläutern die Erfindung.

## Herstellungsbeispiele

### Beispiel 1: Allylbicyclo[2.2.1]hept-5-en-2,3-dicarbonsäure-N-(2′-acryloyloxyethyl)imid

Allylbicyclo[2.2. 1]hept-5-en-2,3-dicarbonsäure-N(2′-hydroxyethyl)imid wird gemäss Beispiel 2 der U.S. 4,728,742 hergestellt.

370,5 g dieser Verbindung und 166,96 g Triethylamin werden in 1200 mi Toluol gelöst und auf 5 °C abgekühlt. Unter starkem Rühren und äusserer Kühlung werden dieser Lösung 135,75 g Acrylsäurechlorid so zugetropft, dass die Temperatur zwischen 4 und 7 °C verbleibt. Danach rührt man das Reaktionsgemisch über Nacht bei Zimmertemperatur, setzt am nächsten Morgen 600 ml deionisiertes Wasser zu und stellt mit 1N-HCl einen pH von 4,7 ein. Man wäscht 2 mal mit 500 ml Wasser, trocknet über $Na_2SO_4$ und destilliert das Toluol am Rotationsverdampfer bei 50 °C und 13,3 Pa ab. Man erhält 406 g Rohprodukt als hellbraunes Oel, was einer Ausbeute von 93,8 d. Th. entspricht.

Diesem Rohprodukt werden 0,5 % Methylenblau als Stabilisator für die Reinigung durch Destillation zugefügt. Kp bei 27 Pa: 180- 190 °C, Ausbeute 380,8 g entsprechend 84,5 % d. Th.

| Analyse: | berechnet für $C_{17}H_{19}NO_4$ | | gefunden |
|---|---|---|---|
| | % C: | 67,76 | 67,49 |
| | % H: | 6,36 | 6,38 |
| | % N: | 4,65 | 4,76 |
| | $n^{D}_{25}$ = | 1,5227 | |
| | $\eta_{25}$ = | 650 mPa•s | |

Die Polymerisation dieses Monomeren verläuft unter Freisetzung von 575,75 J/g. Bei der Differentialthermoanalyse (Mettler TA 3000) wird ein Reaktionsmaximum bei 164° C beobachtet, das der Acrylpolymerisation zuzuordnen ist, sowie zwei andere bei 251 und 295° C, die der Allylnadic-Gruppe entsprechen. Nach der Polymerisation während 6 h bei 250° C erhält man einen klaren zähen Festkörper mit einer Glasumwandlungstemperatur von 322° C.

Beispiel 2: Allylbicyclo[2.2.1]hept-5-en-2,3-dicarbonsäure-N-(2′-methacryloyloxyethyl)imid

123,5 g Allylbicyclo[2.2.1]hept-5-en-2,3-dicarbonsäure-N(2′-hydroxyethyl)imid (gemäss Beispiel 2 der U.S. 4,728,742),
150 g Methylmethacrylat,
0,137 g Hydrochinon
0,082 g Phenothiazin und
1,02 g Natriummethylat
werden unter einer 30 cm langen Kolonne erhitzt, die mit Ringen (d = 3 mm aus Stahldraht gefüllt ist. Bei einer Innentemperatur von 102° C wird ein Destillat mit einer Brechungszahl von 1,4038 erhalten, das 15 % Methanol enthält. Bei 110° C destilliert ein azeotropes Gemisch aus 50 % Methanol und 50 % Methylmethacrylat über ($n^{D}_{25}$ = 1,3768). Nach 2 Stunden währender Umesterung setzt man weitere 1,02 g $NaOCH_3$, nach 3,5 Stunden nochmals 0,3 g zu. Man erhält total 54 g Destillat.

Danach kühlt man ab, versetzt mit 200 g Toluol und schüttelt mit 200 g 0,25N-NaOH aus. Man wäscht 2-mal mit Wasser, trocknet über $Na_2SO_4$ und entfernt das Toluol am Rotationsverdampfer bei 60° C und vermindertem Druck, zuletzt bei 15 Pa. Man erhält 125 g eines gelben Oels (79,3 % d. Th.) folgender Elementarzusammensetzung:

| | berechnet für $C_{18}H_{31}NO_4$ | gefunden |
|---|---|---|
| % C: | 68,55 | 68,35 |
| % H: | 6,71 | 6,73 |
| % N: | 4,44 | 4,49 |

Das Oel wird bei 145-146° C und 0,4 Pa rektifiziert. Destillatausbeute: 73,8 %.
$n^{D}_{25}$ = 1,5211
$\eta_{25}$ = 908 mPa•s

Bei der thermischen Polymerisation werden 90,68 J/g bei einem Reaktionsmaximum von 122° C, und 304,58 J/g bei einem Reaktionsmaximum von 290° C freigesetzt. Nach der Härtung während 6 Stunden bei 250° C beträgt die $T_G$ = 295° C.

Beispiel 3: Allylbicyclo[2.2.1]hept-5-en-2,3-dicarbonsäure-N-(2′-cinnamoyloxyethyl)imid

49,4 g Allylbicyclo[2.2. 1]hept-5-en-2,3-dicarbonsäure-N-(2′-hydroxyethyl)imid (gemäss Beispiel 2 der U.S. 4,728,742)
29,3 g Zimtsäure
0,6 ml 50%ige, wässrige unterphosphorige Säure und
20 ml Xylol Isomerengemisch werden zum Sieden erhitzt. Während 8 Stunden wird bei 160-165° C ein

Xylol-Wasser-Azeotrop abdestilliert. Das Wasser wird abgetrennt, und das Xylol wird zurückgeführt. Nach weiteren 16 Stunden wird mit Sodalösung und Wasser gewaschen, über $Na_2SO_4$ getrocknet, und danach wird das Xylol am Rotationsverdampfer abdestilliert. Es hinterbleiben 75 g (=90 % d. Th.) eines hellgelben Harzes mit einer Viskosität von 2,8 Pa•s bei 80 °C.

| Analyse: | berechnet für $C_{23}H_{23}NO_4$ | gefunden |
|---|---|---|
| % C: | 73,19 | 72,68 |
| % H: | 6,14 | 6,24 |
| % N: | 3,71 | 3,71 |

Beispiel 4: Allylbicyclo[2.2.1]hept-5-en-2,3-dicarbonsäure-N-acryloyloxyimid

Man löst 69,5 g Hydroxylamin-hydrochlorid in Wasser und setzt 204 g Allylbicyclo[2.2.1]hept-5-en-2,3-dicarbonsäure-anhydrid zu. Dann tropft man unter Rühren 80 g 50%ige wässrige Natronlauge zu, erhitzt 1 Stunde auf Rückfluss, destilliert danach alle flüchtigen Anteile ab, nimmt den Rückstand in Toluol auf, filtriert vom ungelösten Kochsalz ab und entfernt das Lösungsmittel am Rotationsverdampfer bei 150 °C und 2000 Pa.

129,2 g dieses Rückstandes (Allylbicyclo[2.2.1]hept-5-en-2,3-dicarbonsäure-N-hydroxylimid) und
65,7 g Triethylamin werden in
470,0 g Toluol gelöst. Diese Lösung wird auf 4 °C abgekühlt. Unter starkem Rühren und unter äusserer Kühlung tropft man 53,4 g Acryloylchlorid so zu, dass die Temperatur zwischen 4 und 6 °C verbleibt. Man lässt über Nacht bei Raumtemperatur rühren, gibt danach 200 ml $H_2O$ zu und stellt mit 60g 1N-HCl einen pH-Wert von 5,4 ein. Nach der Aufarbeitung analog Beispiel 1 erhält man 146,7 g (91 % d. Th.) eines braunen Oels mit folgender Elementarzusammensetzung und Molekulargewicht:

| | berechnet für $C_{15}H_{15}O_4N$: | gefunden |
|---|---|---|
| % C: | 65,92 | 65,80 |
| % H: | 5,53 | 5,62 |
| % N: | 5,13 | 5,13 |
| $\overline{M}_n$: | 273 | 271 (GCP in THF) |
| $\overline{M}_w$: | | 275 |

Nach 12-stündigem Erhitzen einer kleinen Probe wird ein vernetztes Polymer mit $T_G$ = 340 °C erhalten.

Beispiel 5: Allylmethylbicyclo[2.2.1]hept-5-en-2,3-dicarbonsäure-N-(2′,2′-dimethyl-3′-acryloyloxypropyl)imid

54,56 g Allyl-methyl-bicyclo[2.2.1]hept-5-en-2,3-dicarbonsäureanhydrid (hergestellt gemäss Beispiel 2 der U.S. Patentschrift 3,105,839) und
25,79 g Neopentanolamin werden 3 Stunden am absteigenden Kühler auf 130-150 °C erhitzt. Dabei destillieren 4,5 $cm^3$ $H_2O$ ab. Danach rektifiziert man das Hydroxyneopentylimid bei 181-185 °C/13 Pa.
25 g des obigen Reaktionsprodukts und
9,21 g Triethylamin werden in
60 g Toluol gelöst und auf 3 °C abgekühlt. Man tropft unter starkem Rühren und äusserer Kühlung
7,42 g Acryloylchlorid so zu, dass die Temperatur zwischen 3 und 7 °C verbleibt.
Danach lässt man die Temperatur auf 20 °C steigen und rührt über Nacht.

Man arbeitet das Reaktionsgemisch auf, wie dies im Beispiel 1 beschrieben ist, und erhält 29,3 g eines hellbraunen Oels, was einer quantitativen Ausbeute entspricht. Nach der Rektifikation in Gegenwart von 0,2 Methylenblau bei 167-168 °C/100 Pa erhält man 11,35 g (34,2 % d. Th.) einer viskosen Flüssigkeit mit

folgender Elementarzusammensetzung und Eigenschaften:

| | berechnet für $C_{21}H_{27}NO_4$: | gefunden |
|---|---|---|
| % C: | 70,58 | 69,06 |
| % H: | 6,62 | 7,70 |
| % N: | 3,92 | 4,30 |
| $\overline{M}_n$: | 357 | 355 (GPC in THF) |
| $n^D_{25}$ = | 1,5081 | |
| $\eta_{25}$ = | 2,88 Pa•s | |

Polymerisationswärme (DTA): 420 J/g
Reaktionsmaxima: 182° C (Acryl)

$$\left.\begin{matrix}265°C\\315°C\end{matrix}\right\}\ \text{Allylnadic}$$

Glasumwandlungstemperatur des vernetzten Polymeren: 202° C.

Beispiel 6: Allylbicyclo[2.2.1]hept-5-en-2,3-dicarbonsäure-N-(4′-acryloyloxyphenyl)imid

295 g Allylbicyclo[2.2.1]hept-5-en-2,3-dicarbonsäure-N-(4′-hydroxyphenyl)imid (hergestellt gemäss Beispiel 6 der U.S. 4,728,742) und
106,2 g Triethylamin werden in
450 g Toluol gelöst. Dieser Lösung werden
90,5 g Acryloylchlorid unter starkem Rühren und Kühlen bei 8-10° C zugesetzt. Man lässt über Nacht bei Zimmertemperatur ausreagieren, setzt 400 ml Wasser zu, stellt mit 1N-HCl einen pH von 4,0 ein, dekantiert, wäscht noch 2-mal mit je 400 ml $H_2O$ und trocknet über $Na_2SO_4$. Beim Eindampfen der Toluollösung bildet sich ein Kristallbrei, den man kühlt, scharf absaugt und mit Heptan wäscht. Man trocknet die Kristalle bei 40° C im Vakuum und erhält 298,4 g (85,5 % d. Th.) des Acrylates, Fp = 62-65° C.

| Elementaranalyse: | berechnet für $C_{21}H_{19}NO_4$: | gefunden |
|---|---|---|
| % C: | 72,19 | 71,92 |
| % H: | 5,48 | 5,55 |
| % N: | 4,01 | 3,88 |
| $\overline{M}_n$: | 349 | 354 (GPC in THF) |

Ein bei 250° C hergestelltes Polymer hat eine Glasumwandlungstemperatur von 262° C.

Beispiel 7: Allylbicyclo[2.2.1]hept-5-en-2,3-dicarbonsäure-N-[2′-(2″-hydroxy ethoxy)ethyl]imid

81,6 g Allylbicyclo[2.2.1]hept-5-en-2,3-dicarbonsäureanhydrid und
42 g Diglykolamin [2-(2′-Aminoethoxy)ethanol] werden während 4,5 Stunden kondensiert und destilliert (Kp = 196-197° C bei 1,6 Pa). Ausbeute 89,3 g des entsprechenden Hydroxyethoxyethylimids.
25 g des obigen Zwischenprodukts (hergestellt gemäss Beispiel 4 der U.S. 4,728,742),
9,57 g Triethylamin,
7,78 g Acryloylchlorid und
60,0 g Toluol

werden nach dem im Beispiel 1 angegebenen Verfahren umgesetzt.

Aus 30,8 g. Rohprodukt erhält man nach der Rektifikation bei 176-180 °C und 53 Pa 17,02 g (57,4 d. Th.) des Acrylats als hellgelbes Oel mit $\eta_{25}$ = 440 mPa•s.

| Elementaranalyse: | berechnet für $C_{19}H_{23}NO_5$: | gefunden |
|---|---|---|
| % C: | 66,07 | 65,10 |
| % H: | 6,71 | 6,89 |
| % N: | 4,06 | 4,19 |
| $\overline{M}_n$: | 345 | 383 (GPC in THF) |
| $n^D_{25}$ = | | 1,5189 |

Polymerisationswärme: 306,56 J/g

Reaktionsmaxima: 195,4 °C

258,7 °C

303 °C

$T_G$ nach Polymerisation während 6 h bei 250 °C: 167 °C.

Beispiel 8: Allylbicyclo[2.2.1]hept-5-en-2,3-dicarbonsäure-N-(4′-acryloyloxycyclohexyl)imid

150,5 g Allylbicyclo[2.2.1]hept-5-en-2,3-dicarbonsäure-N-(4′-hydroxycyclohexyl)imid (hergestellt durch Umsetzung von Allylbicyclo[2.2.1]hept-5-en-2,3-dicarbonsäureanhydrid mit 4-Aminocyclohexanol gemäss der allgemeinen Vorschrift der U.S. 4,728,742) und

55,7 g Triethylamin

werden in 500 g Toluol gelöst und auf 5 °C abgekühlt. 45,25 g Acrylsäurechlorid werden langsam zugetropft. Die Umsetzung der Edukte und die Aufarbeitung erfolgen wie im Beispiel 1 beschrieben. Man erhält 89 g (50,2 % d.Th.) eines klaren gelben viskosen Harzes.

| Analyse: | berechnet für $C_{21}H_{25}NO_4$: | gefunden |
|---|---|---|
| % C: | 70,96 | 71,14 |
| % H: | 7,09 | 7,21 |
| % N: | 3,94 | 3,65 |
| $\eta_{80°C}$: | 908 mPa•s | |

Anwendungsbeispiele

Beispiel A1:

Ein Gemisch aus 1 Teil Allylbicyclo[2.2.1]hept-5-en-2,3-dicarbonsäure-N-(2′-acryloyloxyethyl)imid (hergestellt gemäss Beispiel 1) und 0,03 Teilen Benzildimethylketal wird als 12 µm dicke Schicht auf ein kupferbeschichtetes Laminat aufgetragen. Die Schicht wird durch ein Negativ mit einer 5 kW Metallhalogenidlampe aus einer Entfernung von 75 cm während 2 Minuten belichtet. Die Entwicklung in Toluol ergibt ein Negativbild. Die thermogravimetrische Analyse des Bildes, d.h. des gehärteten Materials, unter Stickstoff zeigt den Beginn des Gewichtsverlustes bei 395 °C.

Beispiel A2:

Beispiel A1 wird wiederholt, mit Ausnahme, dass nach der Entwicklung des Bildes dieses während 2 Stunden bei 150° C erhitzt wird. Die thermogravimetrische Analyse des Bildes unter Stickstoff zeigt den Beginn des Gewichtsverlustes bei 415° C.

Beispiel A3:

Ein Gemisch aus 1 Teil des Produktes gemäss Beispiel 1,0,03 Teilen 1-Hydroxycyclohexylphenylketon und 0,05 Teilen Allylbicyclo[2.2.1]hept-5-en-2,3-dicarbonsäure-N-benzolsulfonyloxyimid (hergestellt gemäss Beispiel 1 der U.S. 4,709,047) wird als 12 µm dicke Schicht auf ein kupferbeschichtetes Laminat aufgetragen. Die Schicht wird durch ein Negativ mit einer 5 kW Metallhalogenidlampe aus einer Entfernung von 75 cm während 3 Minuten belichtet. Die Entwicklung in Toluol ergibt ein Negativbild. Das entwickelte Bild wird dann während 2 Stunden bei 150° C erhitzt. Die thermogravimetrische Analyse des Bildes unter Stickstoff zeigt den Beginn des Gewichtsverlustes bei 430° C.

Beispiel A4:

Ein Gemisch aus 1 Teil des Produktes gemäss Beispiel 1,2 Teilen Bis[4-(allylbicyclo[2.2.1]hept-5-en-2,3-dicarboxyimidophenyl)]-methan (hergestellt gemäss Beispiel 11 der U.S. 4,515,962) und 0,03 Teilen 2-Methyl- 1-[4-(methylthio)phenyl]-2-morpholinopropan-1-on wird in 2 Teilen Dimethylformamid gelöst. Die Lösung wird als 12 µm dicke Schicht auf ein kupferbeschichtetes Laminat aufgetragen und während 5 Minuten bei 110° C getrockent, wobei ein klebfreier Film entsteht. Der Film wird durch ein Negativ mit einer 5 kW Metallhalogenidlampe aus einer Entfernung von 75 cm während 5 Minuten belichtet. Die Entwicklung in Ethanol ergibt ein Negativbild. Das entwickelte Bild wird dann während 2 Stunden bei 150° C erhitzt. Die thermogravimetrische Analyse des Bildes unter Stickstoff zeigt den Beginn des Gewichtsverlustes bei 180° C (weniger als 10 Gew.%; Verdampfung des restlichen Dimethylformamid-Lösungsmittels) und bei 405° C.

Beispiel A5:

Ein Gemisch aus 1 Teil des Produktes gemäss Beispiel 1,2 Teilen Bis[4-(allylbicyclo[2.2.1]hept-5-en-2,3-dicarboxyimidophenyl)]-methan (hergestellt gemäss Beispiel 11 der U.S. 4,515,962), 0,03 Teilen 2-Methyl-1-[4-(methylthio)phenyl]-2-morpholinopropan-1-on und 0,15 Teilen Allylbicyclo[2.2.1]hept-5-en-2,3-dicarbonsäure-N-benzolsufonyloxyimid (hergestellt gemäss Beispiel 1 der U.S. 4,709,047) wird in 2 Teilen Dimethylformamid gelöst. Eine 12 µm dicke Schicht dieser Lösung wird auf ein kupferbeschichtetes Laminat aufgetragen und während 5 Minuten bei 110° C getrocknet, wobei ein klebfreier Film entsteht. Der Film wird duch ein Negativ mit einer 5 kW Metallhalogenidlampe aus einer Entfernung von 75 cm während 5 Minuten belichtet. Die Entwicklung in Ethanol ergibt ein Negativbild. Das entwickelte Bild wird dann während 2 Stunden bei 150° C erhitzt. Thermogravimetrische Analyse des Bildes unter Stickstoff zeigt den Beginn des Gewichtsverlustes bei 180° C (weniger als 10 Gew.%; Verdampfung des restlichen Dimethylformamid-Lösungsmittels) und bei 405° C.

Beispiel A6:

Ein Gemisch aus 1 Teil des Produktes gemäss Beispiel 1,2 Teilen des Umsetzungsproduktes eines vorverlängerten Epoxidharzes auf Basis von Bisphenol A (Epoxidzahl 1,55 Val/kg) mit Acrylsäure und 0,06 Teilen Benzildimethylketal wird in 2 Teilen Cyclohexanon gelöst. Eine 24 µm dicke Schicht dieser Lösung wird auf ein kupferbeschichtetes Laminat aufgetragen und während 5 Minuten bei 110° C getrocknet, wobei ein Klebfreier Film entsteht. Dieser Film wird durch ein Negativ mit einer 5 kW Metallhalogenidlampe während 2 Minuten aus einer Entfernung von 75 cm belichtet. Die Entwicklung in 1,1,1-Trichlorethan ergibt ein Negativbild. Das entwickelte Bild wird dann während 2 Stunden bei 150° C erhitzt. Die thermogravimetrische Analyse des Bildes unter Stickstoff zeigt den Beginn des Gewichtsverlustes bei 375° C.

Beispiel A7:

Ein Gemisch aus 12 Teilen des Produktes gemäss Beispiel 1,2 Teilen des im Beispiel A6 beschriebenen Umsetzungsproduktes von Epoxidharz mit Acrylsäure, 0,06 Teilen Benzildimethylketal und 0,05 Teilen Allylbicyclo[2.2.1]hept-5-en-2,3-dicarbonsäure-N-benzolsulfonyloxyimid (hergestellt gemäss Beispiel 1 der U.S. 4,709,047) wird in 2 Teilen Cyclohexanon gelöst. Eine 24 µm dicke Schicht dieser Lösung wird auf ein kupferbeschichtetes Laminat aufgetragen und während 5 Minuten bei 110 °C getrocknet, wobei ein klebfreier Film entsteht. Der Film wird durch ein Negativ mit einer 5 kW Metallhalogenidlampe während 2 Minuten aus einer Entfernung von 75 cm belichtet. Die Entwicklung in 1,1,1-Trichlorethan ergibt ein Negativbild. Das entwickelte Bild wird dann während 2 Stunden bei 150 °C erhitzt. Die thermogravimetrische Analyse des Bildes unter Stickstoff zeigt den Beginn des Gewichtsverlustes bei 385 °C.

Beispiel A8:

Das Produkt gemäss Beispiel 8 wird in eine Form gegossen und während 3 h bei 80 °C, 2 h bei 150 °C, 2 h bei 180 °C, 2 h bei 220 °C und 10 h bei 250 °C gehärtet. Das vernetzte Polymer hat die folgenden Eigenschaften:

Tg (TMA): 354 °C
Biegefestigkeit (ISO 178): 55,3 $N/mm^2$
Randfaserdehnung (ISO 178): 1,69 %
Schlagbiegezähigkeit (ISO 179): 2,88 $kJ/m^2$

**Ansprüche**

1. Imide der Formel I

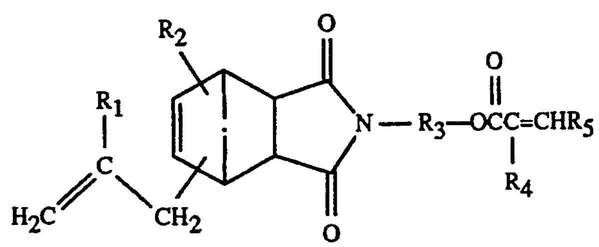

(I),

worin $R_1$, $R_2$ und $R_4$ unabhängig voneinander Wasserstoff oder Methyl bedeuten, $R_3$ eine direkte Bindung oder ein gegebenenfalls durch O-Atome unterbrochener $C_2$-$C_{20}$-aliphatischer, ein ein- oder mehrkerniger $C_5$-$C_{20}$-cycloaliphatischer oder $C_6$-$C_{20}$-aromatischer Rest ist oder für eine Gruppe der Formel II steht

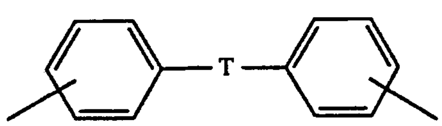

(II),

worin T Methylen, Isopropyliden, CO, O, S oder $SO_2$ bedeutet, und $R_5$ Wasserstoff oder Phenyl ist.

2. Imide nach Anspruch 1, worin $R_1$ und $R_2$ je Wasserstoff bedeuten.

3. Imide nach Anspruch 1, worin $R_4$ Wasserstoff und $R_5$ Phenyl bedeuten.

4. Imide nach Anspruch 1, worin $R_5$ Wasserstoff und $R_4$ Methyl oder vorzugsweise Wasserstoff bedeuten.

5. Imide nach Anspruch 1, worin $R_3$ eine direkte Bindung, ein $C_2$-$C_{10}$-aliphatischer Rest oder eine Gruppe

$$-CH_2CH_2-\!\!\left[-OCH_2CH_2-\right]_m \quad \text{oder} \quad -CH_2\underset{\displaystyle CH_3}{\underset{|}{CH}}-\!\!\left[-OCH_2\underset{\displaystyle CH_3}{\underset{|}{CH}}-\right]_m \quad \text{mit } m = 1$$

oder 2, ein $C_5$-$C_6$-cycloaliphatischer oder $C_6$-$C_{10}$-aromatischer Rest ist oder für eine Gruppe der Formel II steht, worin T Methylen oder Isopropyliden bedeutet.

6. Imide nach Anspruch 1, worin $R_3$ eine direkte Bindung, den Rest

$$-CH_2CH_2-, \quad -CH_2-\underset{\displaystyle CH_3}{\underset{|}{\overset{\displaystyle CH_3}{\overset{|}{C}}}}-CH_2-, \quad -CH_2CH_2OCH_2CH_2-,$$

1,3- oder 1,4-Cyclohexylen oder 1,3- oder 1,4-Phenylen bedeutet.

7. Imide nach Anspruch 1, worin $R_1$, $R_2$ und $R_5$ je Wasserstoff, $R_3$ eine direkte Bindung oder der Rest $-CH_2CH_2-$ und $R_4$ Wasserstoff oder Methyl sind, insbesondere worin $R_1$, $R_2$, $R_4$ und $R_5$ je Wasserstoff bedeuten und $R_3$ der Rest $-CH_2CH_2-$ ist.

8. Verfahren zur Herstellung von Imiden der Formel I nach Anspruch 1 durch Umsetzung eines hydroxylgruppen-haltigen Imids der Formel III

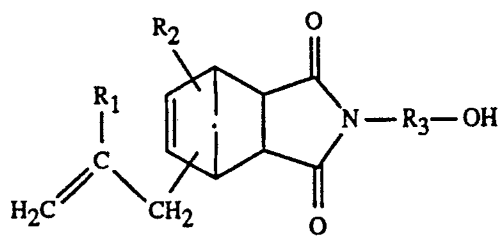

(III),

mit einer Verbindung der Formel IV

$$R_6\overset{\displaystyle O}{\overset{\|}{C}}CR_4=CHR_5 \qquad \text{(IV)},$$

wobei die Reste $R_1$, $R_2$, $R_3$, $R_4$ und $R_5$ die im Anspruch 1 angegebene Bedeutung haben und $R_6$ für OH, Halogen oder $C_1$-$C_4$-Alkoxy steht.

9. Polymere, die dadurch erhältlich sind, dass man ein Imid nach Anspruch 1 während 6 bis 60 Stunden auf eine Temperatur zwischen 180 und 300 °C erhitzt.

10. Polymere, die dadurch erhältlich sind, dass man ein Imid nach Anspruch 1, gegebenenfalls in Gegenwart eines Photoinitiators, mit aktinischer Strahlung belichtet.

11.Verwendung der Imide nach Anspruch 1 als Photoresists.

12. Verwendung der Imide nach Anspruch 1 zur Herstellung von Prepregs.

Patentansprüche für folgenden Vertragsstaat: ES.

1. Verfahren zur Herstellung von Imiden der Formel I

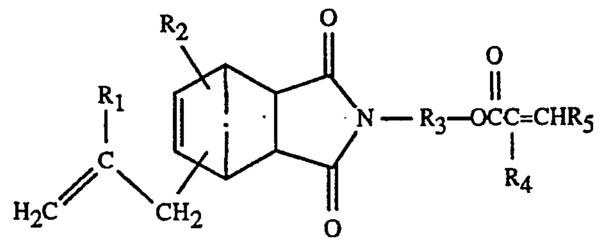

(I),

worin $R_1$, $R_2$ und $R_4$ unabhängig voneinander Wasserstoff oder Methyl bedeuten, $R_3$ eine direkte Bindung oder ein gegebenenfalls durch O-Atome unterbrochener $C_2$-$C_{20}$-aliphatischer, ein ein- oder mehrkerniger $C_5$-$C_{20}$-cycloaliphatischer oder $C_6$-$C_{20}$-aromatischer Rest ist oder für eine Gruppe der Formel II steht

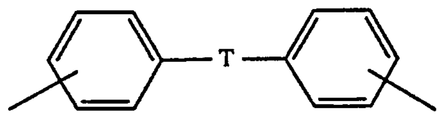

(II),

worin T Methylen, Isopropyliden, CO, O, S oder $SO_2$ bedeutet, und $R_5$ Wasserstoff oder Phenyl ist, durch Umsetzung eines hydroxylgruppen-haltigen Imids der Formel III

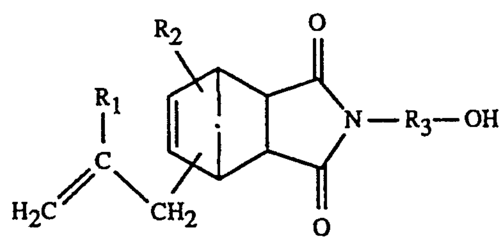

(III),

mit einer Verbindung der Formel IV

$$R_6\overset{O}{\overset{\|}{C}}CR_4=CHR_5 \qquad (IV),$$

wobei die Reste $R_1$, $R_2$, $R_3$, $R_4$ und $R_5$ die oben angegebene Bedeutung haben und $R_6$ für OH, Halogen oder $C_1$-$C_4$-Alkoxy steht.

2. Verfahren nach Anspruch 1, worin $R_1$ und $R_2$je Wasserstoff bedeuten.

3. Verfahren nach Anspruch 1, worin $R_4$ Wasserstoff und $R_5$ Phenyl bedeuten.

4. Verfahren nach Anspruch 1, worin $R_5$ Wasserstoff und $R_4$ Methyl oder vorzugsweise Wasserstoff bedeuten.

5. Verfahren nach Anspruch 1, worin $R_3$ eine direkte Bindung, ein $C_2$-$C_{10}$aliphatischer Rest oder eine Gruppe

$$-CH_2CH_2-[-OCH_2CH_2-]_m \quad \text{oder} \quad -CH_2\underset{CH_3}{\underset{|}{CH}}-[-OCH_2\underset{CH_3}{\underset{|}{CH}}-]_m \ \text{mit } m = 1$$

oder 2, ein $C_5$-$C_6$-cycloaliphatischer oder $C_6$-$C_{10}$-aromatischer Rest ist oder für eine Gruppe der Formel II steht, worin T Methylen oder Isopropyliden bedeutet.

6. Verfahren nach Anspruch 1, worin $R_3$ eine direkte Bindung, den Rest

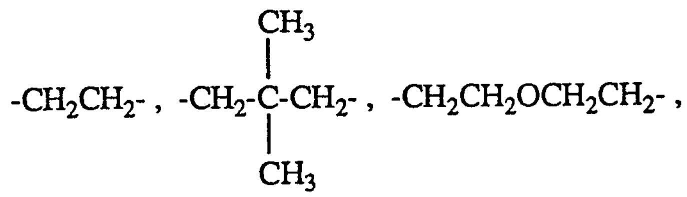

1,3- oder 1,4-Cyclohexylen oder 1,3- oder 1,4-Phenylen bedeutet.

7. Verfahren nach Anspruch 1, worin $R_1$, $R_2$ und $R_5$ je Wasserstoff, $R_3$ eine direkte Bindung oder der Rest $-CH_2CH_2-$ und $R_4$ Wasserstoff oder Methyl sind, insbesondere worin $R_1$, $R_2$, $R_4$ und $R_5$ je Wasserstoff bedeuten und $R_3$ der Rest $-CH_2CH_2-$ ist.

8. Verfahren zur Herstellung von Polymeren durch Erhitzen eines Imids nach Anspruch 1 während 6 bis 60 Stunden auf eine Temperatur zwischen 180 und 300 °C.

9. Verfahren zur Herstellung von Polymeren durch Belichtung eines Imids nach Anspruch 1, gegebenenfalls in Gegenwart eines Photoinitiators, mit aktinischer Strahlung.

10. Verwendung der Imide nach Anspruch 1 als Photoresists.

11. Verwendung der Imide nach Anspruch 1 zur Herstellung von Prepregs.

Europäisches Patentamt

# EUROPÄISCHER RECHERCHENBERICHT

Nummer der Anmeldung

EP 90 81 0149

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | US-A-4 440 850 (J.G. PAUL et al.)<br>* Ansprüche 1,10; Spalte 15, Zeile 10 - Spalte 16, Zeile 2 *<br>--- | 1,10 | C 07 D 209/76<br>C 08 G 73/12<br>C 08 F 22/40<br>G 03 F 7/027 |
| Y | EP-A-0 269 568 (CIBA-GEIGY AG)<br>* Ansprüche 1-6,12 * | 1,10 | |
| A | * Ansprüche 13,14 *<br>--- | 9,11 | |
| A | EP-A-0 190 102 (CIBA-GEIGY AG)<br>* Ansprüche 1-7 *; & US - A - 47 09047 (Kat. D)<br>--- | 1,2,5-8 | |
| A | EP-A-0 166 693 (CIBA-GEIGY AG)<br>* Ansprüche 1-5 *; & US - A - 47 28742 (Kat. D)<br>--- | 1,2,5-7 | |
| A | EP-A-0 152 372 (CIBA-GEIGY AG)<br>* Ansprüche 1-3,7 *; & US - A - 47 42166 (Kat. D)<br>--- | 1,9 | |
| A | EP-A-0 105 024 (CIBA-GEIGY AG)<br>* Ansprüche 1-3,6 *; & US - A - 45 15962 (Kat. D)<br>----- | 1,9 | **RECHERCHIERTE SACHGEBIETE (Int. Cl.5)**<br>C 07 D 209/00<br>C 08 G 73/00 |

Der vorliegende Recherchenbericht wurde für alle Patentansprüche erstellt

| Recherchenort | Abschlußdatum der Recherche | Prüfer |
|---|---|---|
| BERLIN | 11-06-1990 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)